# EUROPEAN PATENT APPLICATION

(11) **EP 2 526 998 A2**
(43) Date of publication of application: **28.11.2012**
(21) Application number: 12180607.9
(22) Date of filing: 14.09.2009
(51) Int. Cl.: A61N 1/36, H05K 5/00, G06F 1/16

(54) **An apparatus for use on a person's lap**

(30) Priority: 01.10.2008 EP 08017293
(62) Divisional of application: 09778518.2
(71) Applicant: CardioLa Ltd., 8404 Wintherthur (CH)
(72) Inventor: Lenz, Christof, 90592 Schwarzenbruck (DE); Seufert, Manuel, 91054 Erlangen (DE); Paul, Christof, 44789 Bochum (DE); Huber, Phil, 8046 Zürich (CH)
(74) Representative: Manitz, Finsterwald & Partner GbR

(57) **Abstract**

An remote controller (2) having at least one of electrical, electronic, pneumatic or hydraulic functions and adapted for use on a person's lap (3), the apparatus having a front side (5) and a rear side (7) generally opposite to said front side there being a wedge (24) at said rear side generally in a central portion thereof and adapted to fit between the upper sides of a person's thighs when in a seated position and wherein the wedge has a surface spaced from said front side and inclined relative thereto such that, when the apparatus is placed on top of a substantially horizontal surface, said front side being parallel to or preferably inclined relative to the substantially horizontal surface.

## Description

The present invention relates to an apparatus having at least one of electrical, electronic, pneumatic or hydraulic functions and adapted for use on a person's lap.

There are many conceivable situations in which it can be convenient for a person to operate an apparatus, especially a control apparatus, which is located on his lap or can alternatively be supported on a surface. For example, an operator of a tower crane may have to sit in a confined space and operate the movement of the jib of the crane, the displacement of the lifting gear along the jib and the lifting and lowering of the hoisting gear. The control apparatus for these functions can comprise one or more joy-sticks connected electrically, pneumatically or hydraulically by flexible lines to the actuators for the crane movements. The operator can place the apparatus on a surface or on his lap to control the crane movements. The apparatus could also communicate electronically e.g. by infrared or radio with corresponding actuators. Similar control apparatuses could be provided in the cockpit of an aeroplane or the cab of a train.

One particular application for control apparatus to which the present invention is principally directed is the control of electrical stimulation units. Here it is envisaged, in accordance with the invention, that a person can be provided with a stimulation unit having leads passing to stimulation electrodes placed on suitable muscles of the person's body and optionally also connected to a heart monitor which may have monitoring electrodes, e.g. connected to the patients chest. An apparatus of this kind is for example described in EP1078649B1.

The control apparatus can be operated by the person or patient to control, for example, the level of stimulation applied to the stimulation electrode via the stimulation unit. When using the apparatus in this way it will be appreciated that the electrical stimulation can cause reflex movements by the person which can make it difficult for him to hold the control apparatus on his lap, which he may wish to do, for example, from the comfort of an armchair or possibly from a wheelchair.

Thus it is a first object of the present invention to provide an apparatus of the initially named kind which is easily held by the person on his lap and cannot be easily dropped even if the person is undergoing reflex movements or is subject to jolting movements, e.g. due to turbulence in an aircraft or wind or load induced movement in a crane.

It is a further object of the invention to provide an apparatus of the initially named kind which can alternatively be operated when placed sitting on a surface.

It is yet a further object of the invention to provide such an apparatus which has a facility for recharging batteries contained there and for communication with further apparatus at a remote location.

Yet another object of the invention is to provide an apparatus of the above mentioned kind which is configured for comfortable use in combination with a stimulation unit and/or in communication with further apparatus.

Another object of the invention is to provide apparatus of the initially named kind which is simple and convenient to use, has significant flexibility in use and which should also be suited in a specific design for use with electrotherapy stimulation apparatus.

The electrotherapy stimulation apparatus should also have a novel design allowing great flexibility is use.

In order to satisfy these objects there is provided, in accordance with a first object of the present invention, an apparatus having at least one of electrical, electronic, pneumatic or hydraulic functions and adapted for use on a person's lap, the apparatus having a front side and a rear side generally opposite to said front side there being a wedge at said rear side generally in a central portion thereof and adapted to fit between the upper sides of a person's thighs when in a seated position and wherein the wedge has a surface spaced from said front side and inclined relative thereto such that, when the apparatus is placed on top of a substantially horizontal surface, said front side is parallel to or preferably inclined relative to the substantially horizontal surface.

In a first position the apparatus is adapted so that it rests, between a person's legs which prevents a potential slipping of the apparatus from the legs, which is a common issue with remote controls such as television remote controls, placed in the same position, and can lead to significant damage to the remote control. In a further position the apparatus provides a further support point, the bottom of the wedge, which is expediently formed such that the front side of the apparatus is at an angle to an essentially flat surface on which the apparatus is placed. This angle between the front side and the bottom of the wedge can range from 0° to 90° and in particular from 10° to 35°. The wedge can be designed such that the angle of inclination is fixed or such that it can be varied.

In a preferred embodiment the apparatus is in the form of a controller for a stimulation unit, said stimulation unit also being adapted to dock onto a docking station to supply electrical power to a battery of the stimulation unit via the docking station, the stimulation unit being optionally adapted to communicate with the docking station and/or a remote controller via respective interfaces and the stimulation unit optionally including at least one of a storage unit, an evaluation unit and a microprocessor.

One or more different devices can be arranged at the front side of the apparatus and can be chosen from a wide variety of appliances, for example, it could be at least one of the following: a joystick, a remote controller, a laptop, a keyboard, a gaming console, a push-button control and a switch control or a television remote control.

Typically laptops are formed with a flat bottom surface, so that when placed on a flat surface they can sit flatly on this surface. However, if a person wants to place a laptop on his lap, the stability given to the system when placed on the flat surface is no longer present. If the person using the laptop on his lap sneezes, for example, the laptop could fall from his lap and break.

Furthermore, the keyboards of laptops are normally disposes essentially parallel to the working surface, while keyboards of desktop computers are inclined at an angle to the working surface for ease of use. In accordance with the present invention, when using a wedge as formed in accordance with the invention, this angle could also be obtained for ease of use and for an increase in the security for the laptop when used on a lap.

The apparatus in accordance with the invention can include at least one of the following items: a battery, a screen, a touch screen, at last one button, at least one interface, at least one dial, a storage unit, an evaluation unit, a microprocessor, a CD drive, a camera, a microphone, a keyboard, a DVD drive and at least one loudspeaker. The storage unit can be provided in the form of a memory, such as a hard disk, a flash drive, a memory card, or the like.

Preferably, the interface is at least one of the following: a wireless interface, a docking interface, a communication interface, a USB interface, a serial interface, a parallel port interface, a firewire interface and a card interface. The wireless interface can be at least one of the following: a Bluetooth interface, a wireless LAN interface, a mobile phone interface, an infrared interface, a UMTS interface, an HSDPA interface, a GSM interface, a radio frequency interface, or the like.

Such a device or apparatus will require electrical power which the battery can only provide for a certain period in time, and therefore requires recharging. In a preferred embodiment of the invention the apparatus can be connected via its first interface, a docking interface, to a docking station. The docking station includes a means for supplying electrical power to the apparatus, for example to charge a battery of the apparatus and/or to provide operating power thereto. The apparatus preferably has at least a second interface from the following group: a wireless interface, a docking interface for an associated unit, a communication interface, a USB interface, a serial interface, a parallel port interface, a firewire interface and a card interface. The wireless interface can be at least one of the following: a Bluetooth interface, a wireless LAN interface, a mobile phone interface, an infrared interface, a UMTS interface, an HSDPA interface, a GSM interface, a radiofrequency interface, or the like.

In a further embodiment, a second apparatus can be connectable to the docking station, this can communicate with both the first apparatus and the docking station via an interface. This second apparatus can include at least one of the following: a battery, a screen, a touch screen, at last one button, at least one interface, at least one dial, a storage unit, an evaluation unit, a microprocessor, a CD drive, a camera, a microphone, a keyboard, a DVD drive and at least one loudspeaker. Preferably, the interface is at least one of the following: a wireless interface, a docking interface, a communication interface, a USB interface, a serial interface, a parallel port interface, a firewire interface and a card interface. Again the wireless interface can be at least one of the following: a Bluetooth interface, a wireless LAN interface, a mobile phone interface, an infrared interface, a UMTS interface, an HSDPA interface, a GSM interface, a radiofrequency interface, or the like.

In a particularly advantageous embodiment the first apparatus can be in the form of a remote controller for a stimulation unit which forms the seconds apparatus. This remote controller can, for example, include at least some of the following items: a touch screen, one or more dials, a docking interface, a wireless communication interface, a card interface, at least one button, an evaluation unit, a microprocessor and a loudspeaker.

Thus, using this remote controller (a first apparatus) it is possible to operate, a stimulation unit (a second apparatus). The stimulation unit is preferably designed to provide a non invasive therapy for patients with chronic heart insufficiency, by stimulating certain muscles in synchronization with the heart beat cycle (especially in the so called counterpulsation mode) via electrical impulses. The stimulation unit is adapted to generate electrical stimulation impulses for the patient and is expediently also adapted to monitor a user's heart rhythm or parameters related thereto such as electrical impulses originating from the person's heart, i.e. or e.g. derived in the manner of an ECG (electrocardiogram).

In a preferred embodiment, the electrical impulses provided by the stimulation unit are biphasic, generally rectangular wave impulses which have a (total) duration of approximately 1 ms to 10% of the total duration of the heart beat, which have an amplitude in the range of 50 to +50 V, and in particular preferably in the range from -30 to +30 V and -45 to +45 V, with a pulse repetition frequency variable in the range from 5 Hz to 1 kHz, and in particular are preferably varied from 450 Hz to 1 kHz, for a typical therapeutic session of 45 minutes. More specifically the stimulation therapy is preferably provided by an apparatus in accordance with the claims of EP 1078649B1, the content of which is incorporated herein by reference.

The electrical impulses provided could also be a train of individual pulses corresponding to a train duration which is usually selected to correspond to a time equivalent from 10 to 25 % of the TQ diastole duration of a human being undergoing treatment, with, for example, 10 individual pulses being included in a pulse train.

Preferably, the biphasic rectangular pulse or pulse train is timed to start at the end of the T-phase of the patient's heart beat cycle.

The electrical impulses are expediently transmitted from the stimulation unit via four cables to four electrodes at four points on the patient's body where they are attached using pads. Two further cables are conveniently connected between the stimulation unit and the patient's body and are part of a heart monitor, to monitor the heart rate of the patient during therapy. The cables provided (typically six but more or fewer as the case may be) can be individual cables, or in a preferred embodiment they can be in the form of a wiring loom adapted for the individual patient or type of treatment and with the length of the individual leads selected so that the respective electrodes can be conveniently placed on the required muscles of the patient's body.

The settings of the stimulation unit can be altered by the remote controller, these setting beings settable either by the dials provided or by data transferred uni-directionally or bidirectionally via said at least one interface and/or processed via the microprocessor and/or stored in the storage unit. Furthermore, the stimulation unit has a heart monitor so that, if the heart monitor of the stimulation unit detects irregularities in the pattern of the heart beat detected, the stimulation unit is adapted to transmit an emergency signal to a remote location, said signal containing information from the GPS sensor and the information on the detected irregularities.

During therapy the stimulation unit, which is approximately the size of a mobile phone, can be transported using a specially adapted belt. The stimulation unit is adapted to be carried by the user using a belt, the stimulation unit being attachable to the belt by one of the following means: a magnet, a hook and loop fastener (such as Velcro™), at least one loop, clip or strap. This enables the therapy to take place either sitting down, or while moving about, i.e. walking, or when lying down.

The stimulation unit (the second apparatus) separately from the remote controller (the first apparatus) is adapted to dock onto the docking station so that the docking station supplies electrical power to a battery of the stimulation unit. The stimulation unit is optionally adapted to communicate with the docking station and/or the remote controller via an interface and includes a memory and an evaluation unit.

For the communication of data, the data can be transferred via interfaces to at least one of the remote controller, the docking station or the stimulation unit. Furthermore, the data can be transferred to and from an apparatus (a third apparatus) at a remote location via the wireless interface. For the communication between the stimulation unit and the docking station and/or the remote controller and/or the third apparatus at the remote location, the stimulation unit has at least one of the following interfaces: a wireless interface, a docking interface, a communication interface, a USB interface, a firewire interface, a serial interface, a parallel port interface and a card interface. When not in use the cables and/or the wiring loom of the stimulation unit can be wrapped around the docking station, for space saving and tidy storage.

The remote controller communicating with the stimulation unit can be adapted to vary the frequency and/or the intensity the electrical stimulations provided by the stimulation unit via the dials or knobs and/or the touchscreen of the remote controller. To monitor the stimulations provided by the stimulation unit, a visual animation of the stimulation can be provided on the touchscreen. In this case each stimulation impulse can be viewed on the touchscreen, this animation can provide visual support for the therapeutic effect for the patient, i.e. can provide visual therapy. For patients who might prefer an acoustic animation over visual animation of the stimulation, for example, due to poor eye sight the possibility can be provided of monitoring the stimulation using a built in loudspeaker system which transmits sound signals in synchronization with the electrical stimulation impulses.

Advantageously, the remote controller is adapted to graphically display the type of electrical stimulation impulses provided by the stimulation unit to the individual patient, i.e. the duration, repetition frequency and the amplitude of the electrical impulses. Moreover, the patients ECG recorded by the stimulation unit can be graphically displayed by the remote controller, for example, to be able to monitor the patients heart rhythm without the need to use an external ECG.

In a further embodiment, the recorded ECG can be displayed on the touchscreen of the remote controller, with the conceptual stimulation pattern being superimposed onto the ECG pattern, to enable a patient and/or a medical practitioner to accurately set the position of the electrical impulses relative to the patient's TQRS cycle via the remote controller prior to commencing with the therapeutic session. This has the advantage that no external devices have to be connected to the patient during the setting of the electrical stimulation. The signal from the ECG can be identified clearly, as no perturbing signals, spurious signals or artifacts are misinterpreted by the ECG, since the setting of the stimulation pattern can be carried out with the remote controller in dependence with the patient's ECG without the stimulation impulses actually taking place. This enables the patient to start his treatment with impulse settings which correspond to his actual TQRS cycle. The patient's actual TQRS cycle can fluctuate depending on the nervosity or which activity he or she has just carried out prior to the treatment. The fact that the theoretical stimulation pattern is superimposed onto the current ECG pattern enables the stimulation impulse pattern of the patient to be set up without the patient being subjected to unnecessary and unwanted pulses.

In a further embodiment, the monitored electrical impulses, such as the ECG pulses, of the patient can be stored in the remote controller and/or in the stimulation unit. Advantageously the remote controller is adapted to display parameters and/or patterns relating to the monitored electrical impulses, i.e. the actual pattern of the monitored electrical impulses, variations and/or irregularities of the electrical impulses can be graphically displayed on the remote controller.

Moreover, variations to the pattern of the monitored electrical impulses can be analyzed, i.e. the pattern of the individual R-R cycles detected can be subjected to a spectral analysis for a given period of time, i.e. the irregularity of the patient's heart beat can be determined, the variations of the heart beat is determined by (e.g.) the R-R peaks can be determined relative to a regular R-R pattern and the frequency of the occurrence of different R-R peaks can be determined. These variations can also be graphically displayed as a pattern on the remote controller. Preferably patterns and/or parameters relating to a period corresponding, for example, to the last 1 to 15 minutes of the patient's heart rate are displayed and most preferably the parameters and/or pattern of the patient's last 500 R-R cycles are displayed on the remote controller.

It is also possible to display the actual stimulation impulses or pattern relative to the actual monitored heart activity. For example, the ECG signal can be determined from the ECG electrodes, the stimulation pulses can be excluded from the ECG pulses by appropriate gating, so that they do not disturb the ECG pattern, but the stimulation pulses which are predicted by the software can still be superimposed on the actual ECG pattern, for example, using different colored displays so that all relevant details are available to the observer, i.e. the patient and/or the medical practitioner (who may be at a remote location).

A card reader implemented at the back of the remote controller, can receive chip cards including different patient parameters and specific treatment parameters. This chip card can be handed out to the patient, if used in a physician's practice or in a hospital, by the nursing staff. Whenever the card is inserted into the patient's device or into a card reader provided in the stimulation unit or in the docking station, all values are then automatically transferred to the stimulation unit and the treatment can commence. This is beneficial to both the patient and the nursing staff, as neither of them has to remember the settings for the patient concerned, and thus are prevented from implementing the wrong treatment settings for a particular patient.

A special super user card can also be used or issued with each remote controller. In contrast to the patient cards which, when inserted into the remote controller can only supply this with a limited set of functions tailored to the requirements of specific treatment of the patient, the super user card enables the setting of all required parameters. This super user card is usually kept by the patient's physician and is used to change and/or update the patient information. The patient is usually only able to change the intensity of the stimulation, i.e. the peak voltages applied to the stimulation electrodes, optionally in dependence on the frequency of the stimulation, but is not otherwise able to alter the individual dependencies or parameters of the type of stimulation provided by the stimulation unit.

The apparatus in accordance with the invention is thus adapted for use in combination with a patient card permitting restricted manipulation and for use in combination with a super user card permitting more comprehensive manipulation, said patient card and said super user card being insertable into a card interface provided at at least one of said remote controller, an associated stimulation unit or an associated docking station.

In a special embodiment, the clinician or physician can limit the numbers of treatments on the patient card with the built in credit meter, so that a patient has to recharge his patient card with credit prior to a next set of therapeutic sessions. This can lead to a possibly required continual update of the patient's treatment parameters in accordance with the physician's findings. These can then be adapted to the monitored performance of the patient's reception to this treatment. This increases the physician's control on the patient's medical parameters. Furthermore, this feature is implemented to ensure that the patient has actually paid for the continued use of the remote controller and, in a case where the patient has borrowed or rented the remote controller, prevents him from using it more than the credited amount of time.

The patient parameters could be transferred via a wireless interface from a third apparatus at the remote location to the remote controller or vice versa. Respective parameters could then be collected and/or be adjusted at this remote location. The wireless interface can be at least one of the following: a Bluetooth interface, a wireless LAN interface, a mobile phone interface, an infrared interface, a UMTS interface, an HSDPA interface a GSM interface, a radiofrequency interface, or the like.

By installing the wireless interface, the remote controller can act as an emergency call system in the case of cardiac arrest or any other heart related problems monitored and detected by the stimulation unit through the electrodes of the monitoring cables. In this case, when certain heart problems are detected, an emergency vehicle can be dispatched to the patient. In this way, the patient's life may be saved as in other scenarios he or she may not be able to call for help.

Furthermore, the patient may not realize that he is in a critical condition which would be detected by the remote controller, and the corresponding emergency measures could be implemented. Furthermore, the patient's health can be detected via a third apparatus from a remote location, so that he does not have to visit the physician for an update on his treatment procedure, but the physician can update this from the remote location even without the patient's direct knowledge of this change in treatment, if the physician deems this necessary. Also, the patient can update his credit via a single phone call and his card can be credited with new treatment credit from the remote location.

The remote controller can be adapted to include a routine monitor, which reminds the patient to perform certain actions, such as the intake of medicine, his daily physical workout, or even to drink water to keep the patient hydrated. The device could carry mechanisms to supply drugs, i.e. tablets from an integrated magazine, i.e. blisters or tablet dispensers, at the time the patient is required to take these, i.e. in the morning, at midday, in the afternoon or in the evening etc.

The remote controller can supply questionnaires relating to the daily or weekly state of health of the patient in the form of a diary, and either store these in the storage unit or transmit these directly to the remote location, for further analysis by a physician. It should be noted that the stimulation unit can, if necessary, be used for prolonged periods of time without interruption for months on end or at least for several hours a day for a prolonged period.

The remote controller can be adapted to also serve as a remote control for other devices in addition to the stimulation unit such as a TV, a DVD player, a radio or a music playing system.

The remote controller can act as an internet or cable TV interface, via the wireless interface, such that the patient can perform his or her treatment at any location he or she desires, i.e. while watching television or surfing the internet.

In a particularly advantageous embodiment the stimulation unit is adapted to provide electrical stimulation and pulses for a person and the stimulation unit is adapted to monitor the person's heart rhythm or parameters related thereto, such as the electrical impulses originating from his heart.

In a further embodiment the stimulation unit can be adapted to provide a heart load reduction of a living organism in accordance with WO 01/13990 A1. This discloses an apparatus for treating a mammal or other living organism having a heart and a peripheral vascular system to achieve a heart load reduction, the organism having a pulse rate and a systolic pressure resulting from the action of the heart, the method and apparatus involving the steps of measuring the heart rhythm, producing pressure pulsations in the peripheral vascular system by a non-invasive or invasive method in synchronization with the heart rhythm in the counterpulsation mode and varying at least one parameter of an input system generating the pressure pulsations to produce an optimised reduction in at least one of the pulse rate and the systolic pressure and hereby a net reduction in the heart load, the heart load being a function of the pulse rate and the systolic pressure.

In a further embodiment the stimulation unit can be adapted to provide electrical stimulation to a muscle or group of muscles of a person or other mammal in accordance with WO 2005/0044372 A1. The electrical stimulation comprises electrical pulses, the electrical stimulation having parameters comprising at least some of an amplitude, a pulse repetition frequency, a duration of the pulse or group of pulses and a time offset relative to a predicted end of a T-wave of an electrocardiogram derived from the person or mammal. This apparatus is characterized in that the electrotherapy apparatus is adapted to vary at least one of said amplitude, said pulse repetition frequency, said duration and said offset in accordance with a predetermined pattern, or randomly, within pre-specified limits in the course of a treatment extending over many heart cycles, typically over more than 15 minutes.

In a further embodiment the apparatus can be for the cardio-synchronized stimulation of skeletal or smooth muscle, but excluding the heart muscles, in a counterpulsation mode of a patient having a heart and a cardiovascular system in accordance with WO 2006/053596 A1. The apparatus comprises at least one active electrode and at least one passive electrode adapted for attachment to said patient, a signal processor having an associated configuration input for varying at least a time delay associated with counterpulsation mode stimulation, a sensing system for sensing information relating to the performance of the patient's heart and for transmission of information signals to said signal processor, said signal processor being adapted to produce control signal information relating to stimulation signals to be applied to said at least one active electrode in a counterpulsation mode, a stimulation signal generator associated with said active electrode for generating stimulation signals, wireless transmission means for transmitting said control signal information from said signal processor to said stimulation signal generator whereby said stimulation signal generator applies stimulation signals to said active electrode in a counterpulsation mode in accordance with said signal information.

Further advantageous embodiments of the invention and preferred apparatus for carrying out the method of the invention are set forth in the appended subordinate claims and are described in connection with the accompanying drawings.

The present invention will now be explained in more detail in the following with reference to preferred embodiments and to the accompanying drawings in which are shown:
- Fig. 1: an example of the apparatus in accordance with the invention,
- Fig. 2: a docking station in accordance with the invention,
- Fig. 3: a stimulation unit in accordance with said invention,
- Fig. 4A: a perspective view of the apparatus in accordance with the invention as seen from the front and from above,
- Fig. 4B: a perspective view of the apparatus in accordance with the invention, as seen from the front and from below,
- Fig. 4C: a bottom view of the apparatus in accordance with the invention,
- Fig. 5A: a perspective view of the apparatus in accordance with the invention as seen from the above and from the rear,
- Fig. 5B: a rear view of the apparatus in accordance with the invention,
- Fig. 5C: a side view of the apparatus in accordance with the invention,
- Fig. 6A: a schematic representation showing how the stimulation unit in accordance with the invention can be mounted on a belt,
- Fig. 6B: a schematic representation shows where the electrical pads of the stimulation unit in accordance with the invention can be placed,
- Fig. 6C: a schematic representation of how the apparatus in accordance with the invention can be adjusted with the electrical contacts of the stimulation unit attached to the patient,
- Fig. 6D: a schematic representation of the apparatus of the invention and of the stimulation unit in accordance with the invention with both simultaneously docked at the docking station,
- Fig. 7A: a schematic representation of the apparatus in accordance with the invention,
- Fig. 7B: a schematic representation of the apparatus in accordance with the invention viewed from below,
- Fig. 7C: a schematic representation of the apparatus in accordance with the invention viewed from behind,
- Fig. 7D: a schematic representation of the apparatus in accordance with the invention being held in a person's hands,
- Fig. 7E: a schematic representation of the apparatus in accordance with the invention positioned between a person's legs, while being adjusted,
- Fig. 8: shows a schematic block diagram of the remote controller in accordance with the invention,
- Fig. 9: shows a schematic block diagram of the stimulation unit in accordance with the invention,
- Fig. 10: shows a schematic block diagram of the docking staion in accordance with the invention,
- Fig. 11: shows a schematic diagram illustrating a typical electrocardiogram,
- Fig. 12A: shows a spectrum of the variations of a patient's heart rate (bpm), and
- Fig. 12B: shows variations of the arrhythmia of the bpm spectrum of Fig. 12A.

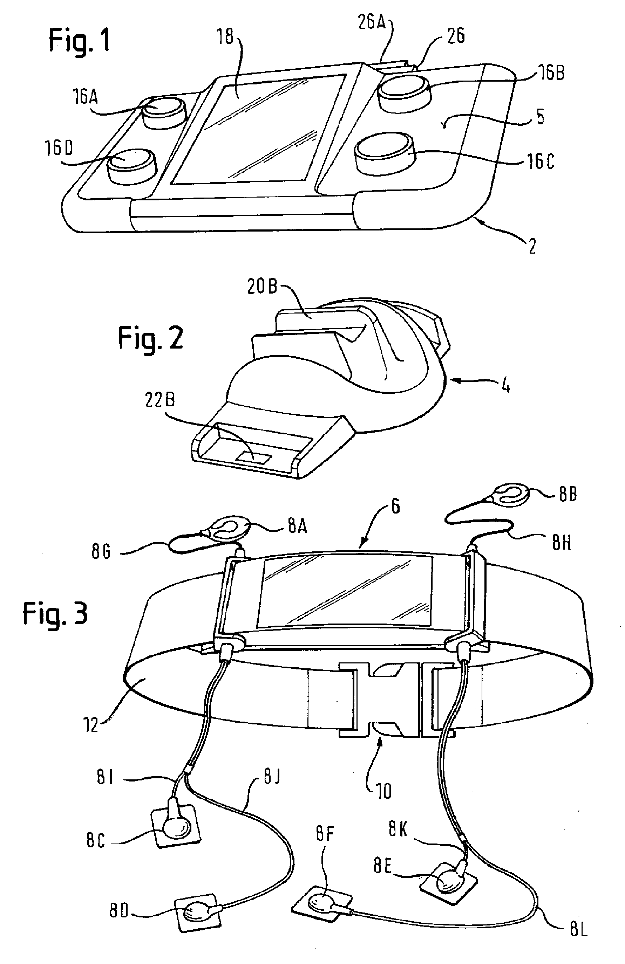

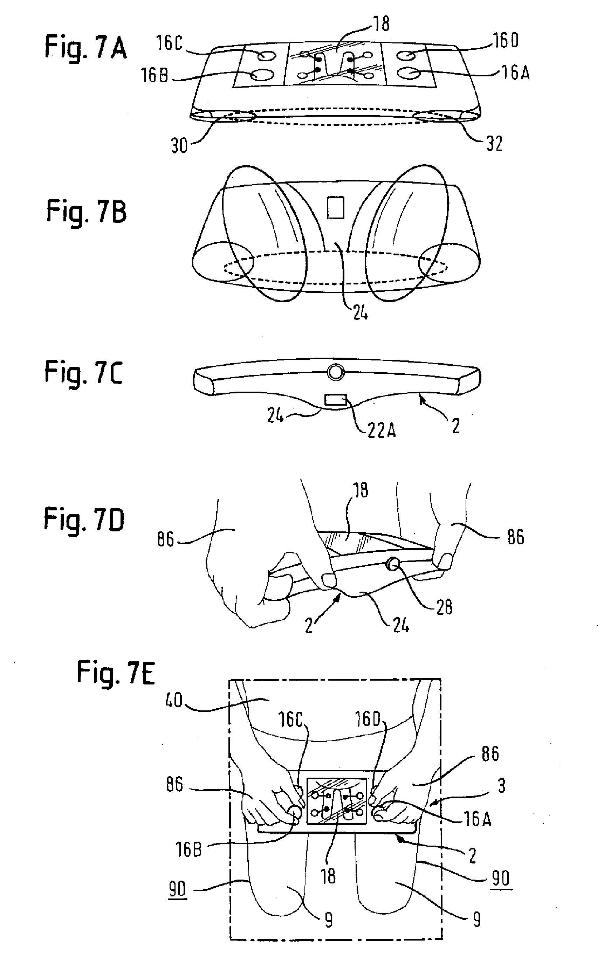

Fig. 1 shows an apparatus 2 for use on a person's lap 3 (Fig. 7E), the apparatus 2 having a front side 5 and a rear side 7 generally opposite to said front side 5. A wedge 24 is provided at said rear side 7 generally in a central portion thereof and is adapted to fit between the upper sides 9 of a person's thighs (see Fig. 7E) when in a seated position. The wedge 24 has a surface spaced from said front side 5 and inclined relative thereto such that, when the apparatus 2 is placed on top of a substantially horizontal surface, said front side 5 is inclined relative to the substantially horizontal surface at an angle convenient for the user. A touchscreen 18 can be seen on the front side 5 to monitor and/or to adapt the intensity and/or the frequency of the electrical stimulation controlled by the four dials 16A, 16B, 16C and 16D and applied to the stimulation electrodes 8C, 8D, 8E, 8F of the stimulation unit 6 (Fig. 3).

The apparatus 2, shown in Fig. 1 does not necessarily have to be in the form of a remote controller 2 for a stimulation unit 6, it could be at least one of the following, a joystick, a laptop, a keyboard, a gaming console, a push-button control and a switch control or a television remote control.

If used as a remote controller 2 for a stimulation unit 6 in accordance with the invention, the settings of the stimulation unit 6 can be altered by the remote controller 2. The settings can be set either by the dials 16A, 16B, 16C, 16D or by data transferred uni-directionally or bidirectionally to the stimulation unit 6 via the at least one interface 22A, 26, 46 of the remote controller 2 and/or processed in the evaluation unit 50 and/or the microprocessor 48 and/or stored in the storage unit 42.

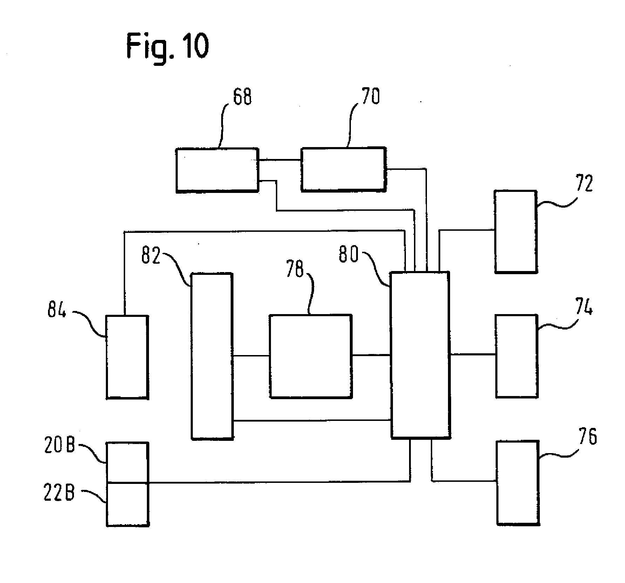

Fig. 2 shows a docking station 4 in accordance with the invention, with the apparatus 2 of Fig. 1, in this example a remote controller 2 for a stimulation unit 6, being connectable to the docking station 4 via a docking interface 22B. The docking station 4 includes means for supplying electrical power to said apparatus 2 of Fig. 1, for example, to charge a battery of the apparatus 2 in accordance with Fig. 1 and to provide operating power thereto, or to communicate with the remote controller 2. The docking station 4 has at least one further interface selected from a group consisting of a wireless interface 72, a docking interface 20B, a communication interface 74 and a card interface 76 (see Fig. 10).

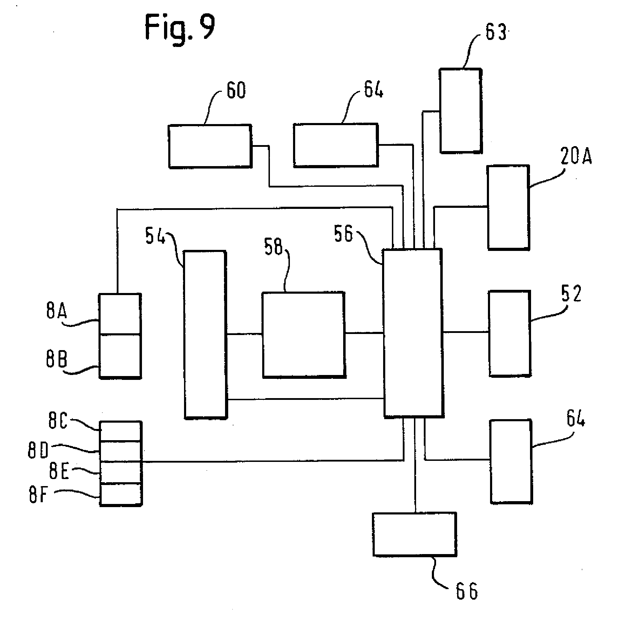

Fig. 3 shows the stimulation unit 6 in accordance with the invention. The stimulation unit 6 is able to connect to the docking station 4 which supplies electrical power to the battery 40 (see Fig. 9) of the stimulation unit 6 via the docking interface 20B; 20A. The stimulation unit 6 is optionally adapted to communicate with the docking station 4 and/or the remote controller 2 of Fig. 1 via at least one further interface 52, 66 (see Fig. 9) and including a storage unit 54, an evaluation unit 56 and a microprocessor 58 (also shown in Fig. 10).

The stimulation unit 6 in accordance with Fig. 3 thus has at least one of the following: a wireless interface 52 (see Fig. 9), a docking interface 20A (see Fig. 9) for mating with interface 20B of the docking station, a communication interface 66 (see Fig. 9) and a card interface (not shown). Furthermore, the stimulation unit 6 is adapted to provide electrical stimulation impulses for the patient via the stimulation electrodes 8C, 8D, 8E, 8F. The stimulation unit 6 is adapted to monitor the user's heart rhythm or parameters related thereto, using the electrodes 8A, 8B, attached to the cables 8G, 8H. The electrical impulses provided by the stimulation unit 6 are generally biphasic, rectangular wave impulses which have a duration of approximately 1 ms, which have an amplitude in the range of -50 to +50 V, and in particular preferably of - 30 to +30 V and -45 to +45 V, and a pulse repetition frequency variable in the range from 5 Hz to 1 kHz, and in particular are preferably varied from 450 Hz to 1 kHz.

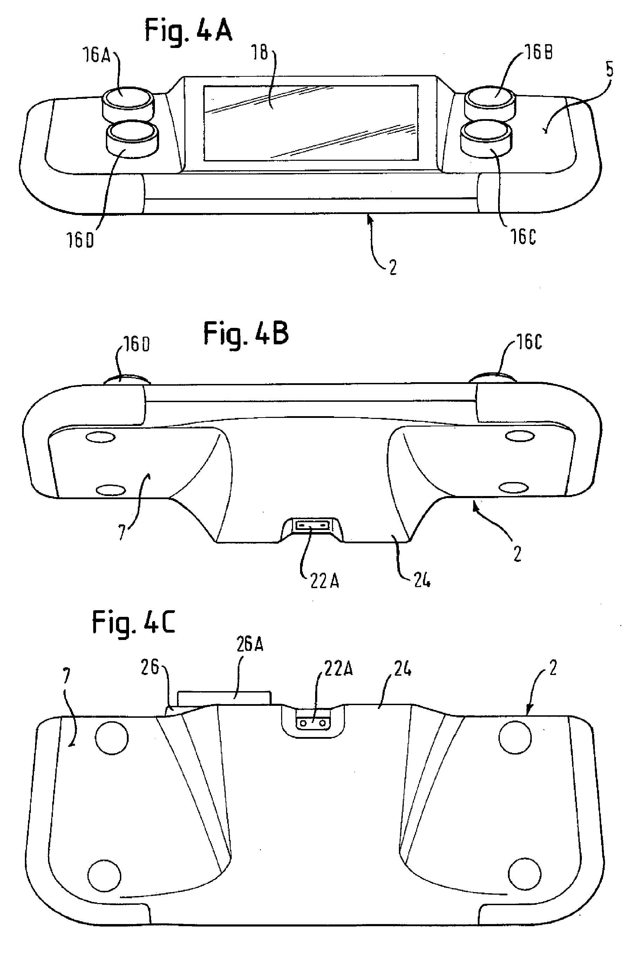

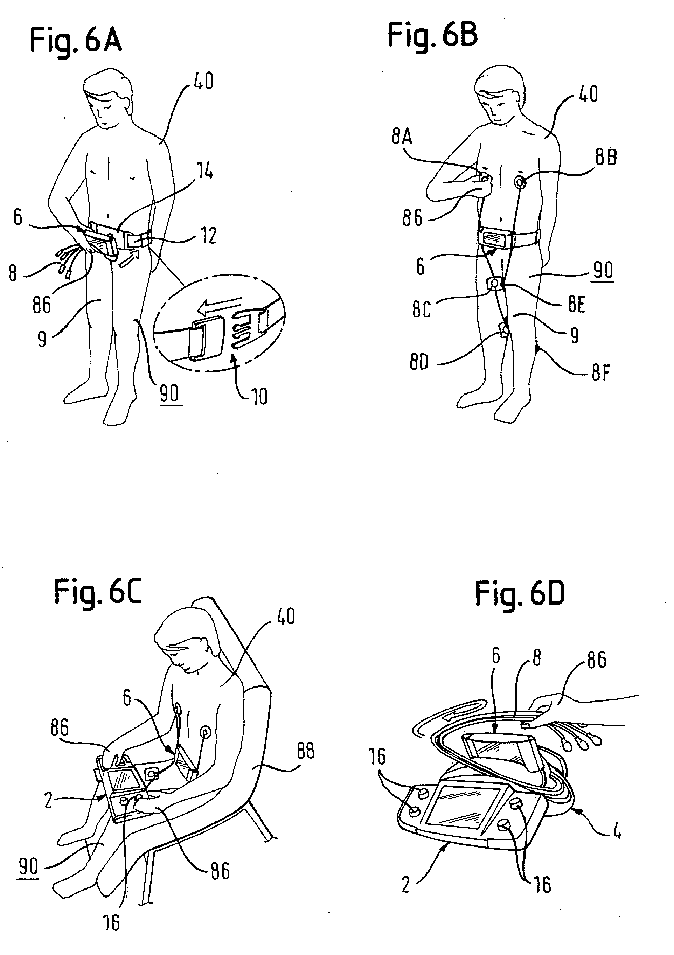

Fig. 4A shows a view of the front side 5 of the remote controller 2 in accordance with the invention. It shows the 4 dials 16A, 16B, 16C, 16D and the touchscreen 18. Fig. 4B shows a perspective view of the apparatus 2 in accordance with the invention as seen from the front and from below. The wedge 24 and the rear side 7 can clearly be seen in Fig. 4B. This three dimensional wedge 24 is formed such that it has a generally central portion and is adapted to fit between the legs 90 of a person 40 (see Fig. 6A) and thus conforms generally to the front and inner sides of his thighs 9 (see Fig. 7E). Furthermore, a cutout is provided at the bottom of this wedge and is formed as an interface 22A to mate with the docking interface 22B. This cutout is thus the docking interface 22A for the apparatus 2. It includes means for applying electrical power to the apparatus 2 in accordance with the invention from the docking station 4, to either charge the battery of the remote controller 2, or to transfer data from the docking station 4 to the remote controller 2.

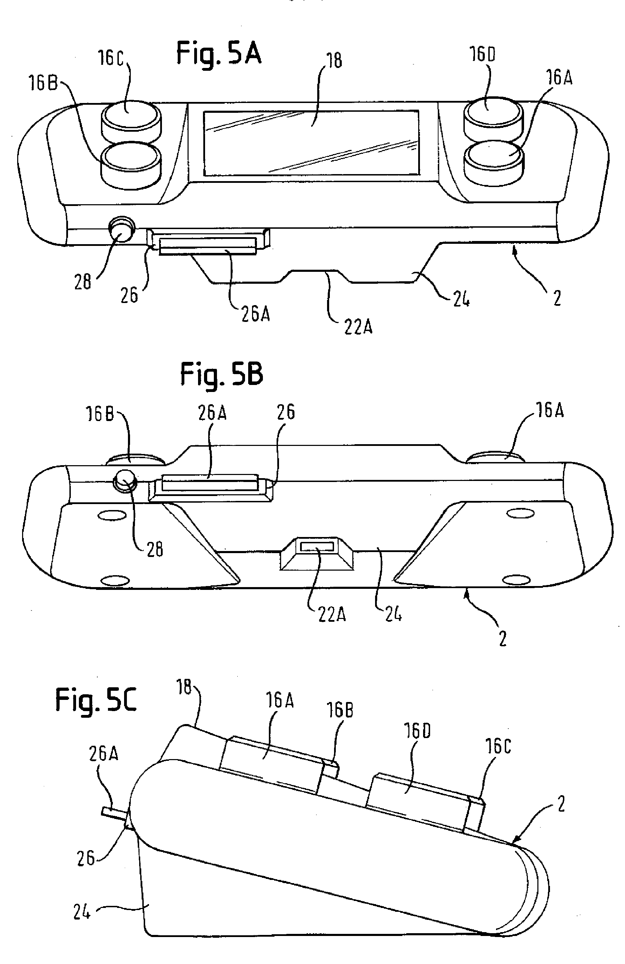

Fig. 4C shows a view of the rear side 7 of the apparatus 2 from below in which the cutout for using the docking interface and a card 26A inserted into the card reader 26. This apparatus 2 is operational when the power button 28 (Fig. 5A) has been pressed. The remote controller 2 can be operated in at least two positions, typically; however, the first of the positions is on the lap 3 (see Fig. 7E) of a person 40, and the second is on a table, or any other essentially flat surface. In the position shown in Fig. 6C, the wedge 24 is arranged in such a way that it rests between the patient's 40 legs, on top of his thighs 9, which avoids potential slipping of the unit from the legs, a common issue with, for example, TV remote controls placed in the same position. In the position shown in Fig. 5C the bottom side of the wedge 24 provides a support which, supports the unit in a stable position on a horizontal surface. Additionally the wedge 24 lifts the unit relative to a horizontal surface on which it rests and creates an optimized viewing angle for the patient 40. Furthermore, the apparatus 2 can be more easily picked up with the hands 86 of the patient 40 (see Fig. 6A) when resting on a table.

Fig. 5A shows a perspective view of the apparatus 2 from the top and from the rear. A card 26 A inserted into the card reader 26 can be seen as well as the four dials 16A, 16B, 16C and 16D, the touchscreen 18 a power button 28 and the cutout 22A for the docking interface. Fig 5B shows a view from the rear of the apparatus 2, where the triangular shape of the wedge 24 is clearly visible. The cutout 22A for the docking interface can also be clearly seen. The two dials 16A and 16B for the control of the frequency and/or the intensity of stimulation of the stimulation unit 6 are also shown in Fig. 5B.

Fig. 5C shows a view of the apparatus 2 from one side. Clearly seen is the wedge 24, providing an angle between the surface on which the apparatus 2 is placed and the front side of the apparatus 2. In general this angle is between 0° and 90° and in a preferred arrangement between 5° and 35°. Also indicated are the touchscreen 18 and the dials 16A, 16B, 16C and 16D.

Fig. 6A shows a person 40 in the act of placing the stimulation unit 6 on the belt 12 provided for the stimulation unit 6. In the enlarged detail view to the right of the person 40, the belt buckle 10 of the belt 12 can be seen through which the belt 12, is fastened about the persons 40 body. The stimulation unit 6 is adapted to be carried by the user 40 using the belt 12. The stimulation unit 6 can be attached to the belt 12 by one of the following means: a magnet, a hook and loop fastener (such as Velcro™), at least one loop, clip or strap.

In Fig. 6B one can see the person 40 applying the electrical contact 8A to his body 40. This electrical contact 8A is applied along with the electrical contact 8B to measure the electric impulses from the patient's 40 heart. Also shown are the electrodes 8C, 8D, 8E and 8F, which in this case are used to apply the electrical stimulation to the person's muscles. There is no muscle group, which can not be stimulated by the stimulation unit 6, however, the preferred muscle groups stimulated are, the calf muscles, the inner thigh muscles (as shown here) and the outer thigh muscles.

Fig 6C shows schematic illustration of a person 40 seated in a chair 88. In this the user 40 is operating the remote controller 2 which is securely positioned, due to the wedge 24 placed between his legs 90, to monitor and/or control the intensity and/or the frequency of the stimulation, and, via the applied stimulation, his heart rhythm.

Fig. 6D shows the remote controller 2 and the stimulation unit 6 docked at the docking station 4. In this situation, the batteries 60 of both the remote controller 2 and the stimulation unit 6 can respectively be recharged, and the two devices can be stored securely due to the cut outs provided at the docking station 4. Furthermore, the cables 8 of the stimulation unit 6 can be stored in or at the docking station 4.

Fig. 7A shows the remote controller 2 with a display of the stimulation applied to the person's legs being shown on the touchscreen 18. The intensity and/or frequency can be controlled by the dials 16A, 16B, 16C and 16D. This can be done either by arranging the dials such that a change in peak voltage applied to the stimulation electrode is always accompanied by a change in the pulse repetition frequency of the electrical signals forming each separate stimulation impulse or in such a way that each dial can change the peak voltage or the pulse repetition frequency by specifying the desired parameter by use of the touchscreen 18. Fig. 7A shows the slightly curved profile of the remote controller 2 between the two points 30, 32 as also visible in Fig. 5A.

Fig. 7B shows a view from the bottom of the remote controller 2 in which, the wedge 24 can be clearly seen. The wedge 24 is shaped so that it can sit between a person's legs 90 and be operated without the danger of the remote controller 2 placed on top of the wedge 24 falling from the lap 3. That is to say the wedge has side faces that are generally rounded and anatomically shaped complementary to the upper inner side portions of a typical person's thighs 9.

Fig. 7C shows a view from the front end of the remote controller 2, in which the wedge 24 is clearly pronounced to illustrate the fact that when this is placed between the patient's legs 90, it can not fall to the left or the right of the person's legs 90. Fig. 7D shows how the remote controller 2 is shaped such that it can be easily gripped by the hands 86 of a person or user 40.

Fig 7E shows schematic illustration of a person 40 in a chair 88. In this the user 40 is operating the remote controller 2 securely, due to the wedge 24 placed between his legs 90, to monitor and/or control either the intensity or the frequency of the stimulation, and/or his heart rhythm via the stimulation unit 6, using the dials 16A and 16B. The touchscreen 18 schematically shows the electrodes 8C, 8D, 8E, and 8F applied to 4 points of contact at the patient's legs 90. If viewed in time, then one would see the stimulation of these 4 points when they are stimulated.

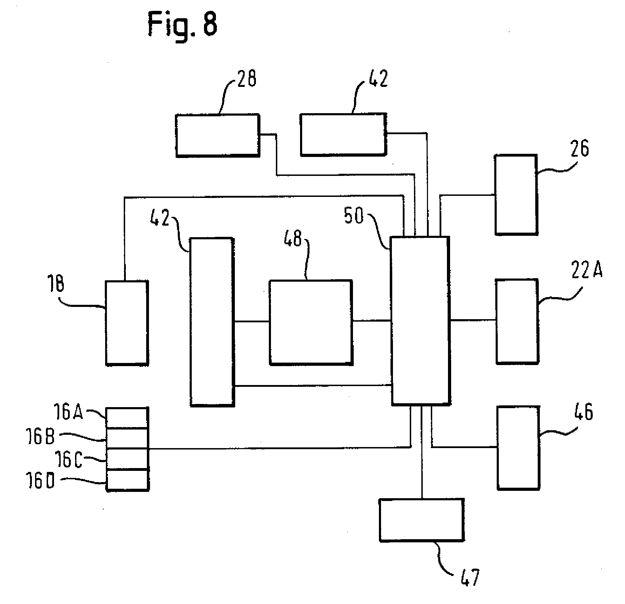

Fig. 8 shows a schematic block diagram of part of the contents of the remote controller 2. At the heart of the remote controller 2 an evaluation unit 50 is communicatively connected to a microprocessor 48 and a storage unit 42. The remote controller can be switched on or off using the button 28 connected to the evaluation unit 50. On starting the remote controller 2, the touchscreen 18, the loudspeaker 42, the docking interface 22A, the card interface 26, the wireless interface 46, the dials 16A, 16B, 16C, 16D, the microprocessor 48, the storage unit 42 and the evaluation unit 50 are put into an active mode. In this active mode, the functions of the remote controller 2 described above can be executed.

Fig. 9 shows a schematic block diagram of part of the contents of the stimulation unit 6. At the heart of the stimulation unit 6 an evaluation unit 56 is communicatively connected to a microprocessor 58 and a storage unit 54. The stimulation unit 6 can be switched on or off using the button 64 connected to the evaluation unit 56. On starting the stimulation unit 6, the electrodes 8A, 8B, 8C, 8D, 8E, 8F, the docking interface 20A, the wireless interface 22A, the storage unit 54, the evaluation unit 56, the microprocessor 58, the battery 60, the credit meter 63, the GPS sensor 64 and the communication interface 66 are put into an active mode. In this active mode, the functions of the stimulation unit 6 described above can be executed.

Fig. 10 shows a schematic block diagram of part of the contents of the docking station 4. At the heart of the docking station 4 an evaluation unit 80 is communicatively connected to a microprocessor 78 and a storage unit 82. The docking station 4 can be switched on or off using the button 68. The button is optionally connectable to either the power supply and/or it is connected to the evaluation unit 80. On starting the docking station 4, the docking interfaces 20B, 22B, the card interface 76, the wireless interface 72, the communication interface 74, the microprocessor 78, the storage unit 82 and the evaluation unit 80 are put into an active mode. In this active mode, the functions of the docking station 4 described above can be executed.

Via its touchscreen 18 the remote controller 2 in accordance with Fig. 1 could also provide visual animation of the stimulations provided by the stimulation unit 6 in accordance with Fig. 3. For example, one could monitor the stimulation impulses provided to the legs via the stimulation electrodes 8C, 8D, 8E, 8F attached to the cables 8I, 8J, 8K, 8L. The animation is intended to help the patient 40 visualize the treatment process and to provide visual support for the therapeutic effect when the patient 40 is being treated for heart problems. Furthermore, via the loudspeakers 42 (Fig. 8) provided for the remote controller 2 in accordance with Fig. 1, an acoustic animation and stimulation is provided the sounds generated can either be synchronized with the stimulation impulses, or alternatively a rhythmic sound could be implemented in a way that the patient's 40 attention is drawn to a potential arrhythmias of his heart beat. The patient's 40 awareness of such arrhythmias could potentially influence the therapeutic effect.

The card interface 26 integrated into the remote controller of Fig. 1 could be for a patient chip card 26A which includes the treatment parameters of the patient 40 which can then be transmitted via a wireless interface to the stimulation unit 6 to set the so called k-factors and the parameters of the treatment required by the patient 40 depending on the what his or her physician has decided on as being the best course of action for the patient 40. The k-factors relate to the ratio of systolic and diastolic phases of operation of the heart as used in the Bazett relationship which influences the timing of the stimulation impulses in a patient dependent manner. This chip card 26A can be handed out to the patient 40. Whenever the patient 40 inserts this card 26A into the card interface 26 of his remote controller 2, all or values are automatically transferred and implemented. The card interface 26 implemented into the remote controller 2, could also be implemented into either the docking station 4 or the stimulation unit 6 if required.

The patient card 26A inserted into the card interface 26 permits the patient to perform certain restricted manipulations of the settings of the stimulation unit 6. Furthermore a special a super user card 26A can be provided, which permits more comprehensive manipulations. The patient card 26A and the super user card 26A can both be inserted into the card interface 26 which is provided, at least at the said remote controller 2, or at an associated stimulation unit 6 or at an associated docking station 4. The patient card 26A can be credited with credit. A credit meter 47, 63, 84 implemented into either the remote controller 2, the docking station 4 or the stimulation unit 6, can be used to debit credit from the patient card 26A in order to account for the number or duration total of treatments the patient 40 has received.

The remote controller 2 can act as telemetric patient hub allowing other devices to connect via a wireless interface, for example a Bluetooth interface, and transmit this data uni-directionally or bidirectionally to another apparatus such as a monitoring computer at a remote location. The remote controller 2 can remind the patient 40 to take certain actions, for example, to take his medicine, to do a physical workout, to keep the body hydrated by drinking etc, by using a routine monitor. The routine monitor can also be implemented into either the docking station 4 or the stimulation unit 6 if required.

A tablet dispenser can be implemented into either the remote controller 2 or the docking station 4 or indeed the stimulation unit 6, to administer tablets or drugs, e.g. packaged in blister form to the patient 40.

The remote controller 2 can supply questionnaires related to the daily or weekly state of health of the patient 40 using a diary, and either store these in the storage unit 42 of the remote controller 2 and/or upload these to an apparatus such as a monitoring computer at a remote location for storage and/or inspection by the patient's physician.

The interfaces of the remote controller 2, the docking station 4 and the stimulation unit 6 can be at least one of the following, a wireless interface 46, 52, 72, a docking interface 20A, 20B, 22A; 22B, a communication interface 66, 74, a USB interface, a serial interface, a parallel port interface, a firewire interface and a card interface 26, 76. Moreover the wireless interfaces if used are at least one of the following, a Bluetooth interface, a wireless LAN interface, a mobile phone interface, an infrared interface, a UMTS interface, an HSDPA interface a GSM interface, a radiofrequency interface, or the like.

As the stimulation unit 6 is provided with a heart beat monitor, it can detect whether the patient 40 is going into cardiac arrest, having a heart attack or suffering some form of cardiac distress. In a life threatening situation, the stimulation unit 6 is adapted to act as an emergency call system, and an ambulance can be dispatched to the patient's whereabouts, detected by a GPS sensor 62 implemented into the stimulation unit 6 in accordance with the invention. Having detected the irregularities in the pattern of the heart beat, the stimulation unit 6 can transmit an emergency signal to a monitoring facility at a remote location, the emergency signal containing information from the GPS sensor 62 and the information on the detected irregularities. The transmitted emergency signal can also be transmitted from remote controller 2 or the docking station 4.

The remote controller 2 can act as a remote control for other appliances. If the patient 40 has a TV or a home music system or a home cinema system then it can be programmed to act as a remote control through the wireless interface 46, the microprocessor 48 and the corresponding software, for these other devices, while the patient 40 is undergoing his or her therapy.

The touchscreen 18 implemented into the remote controller 2 can not only display the operation of the stimulation unit 6, but the microprocessor 48 can also be programmed in such a way, that the remote controller 2 can act as an internet or TV interface controllable through the touchscreen 18. Other applications and extensions of the wedge 24 in accordance with Fig. 1 can be envisaged. Thus any device having the wedge can be operated on the lap, for example laptops, keyboards, or remote controls for diverse applications. The apparatus can furthermore be designed in any way so that it can be folded away and carries additional interfaces, i.e. sockets, plugs, lights or loudspeakers.

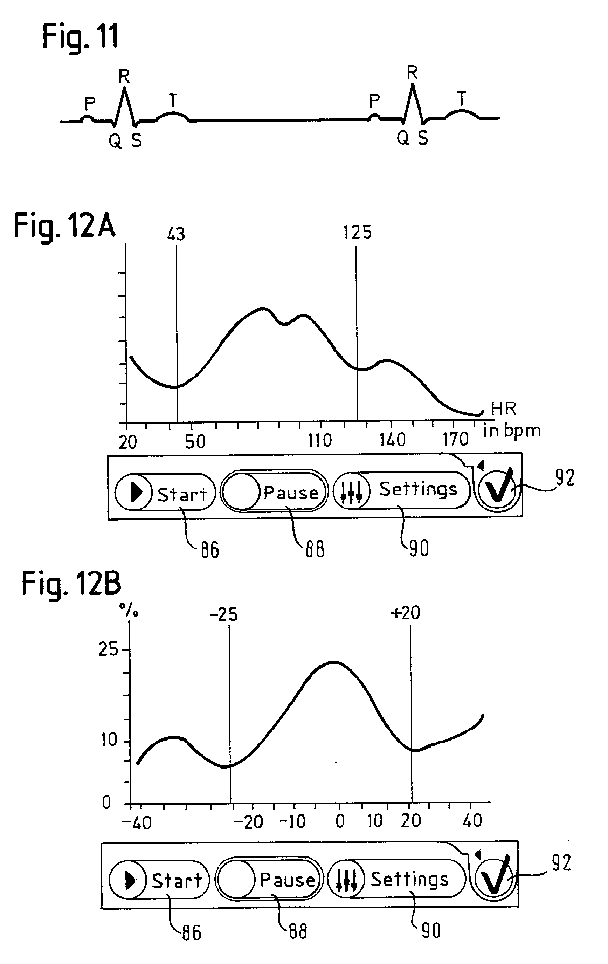

Fig. 11 shows a schematic diagram illustrating a typical electrocardiogram (ECG), with the different deflections P, Q, R, S and T being present in a typical ECG spectrum, as is well known to the person of ordinary skill in the art. The R-R cycle, i.e. the time for two consecutive R-R peaks to occur defines a person's heart rate. Depending on the person and his current physical activity, the heart rate can vary from ∼ 40 bpm to ∼220 bpm. Typically 500 R-R cycles elapse in a period of 5 to 9 minutes for a human being at rest.

The stimulation unit 6 measures each individual R-R pulse separation distance. From this separation distance the stimulation unit 6 and/or the remote controller 2 calculates the actual TQRS cycle and estimates when the next pulse of the stimulation unit 6 is to be transmitted to the patient via the electrodes relative to the measured R-R cycle. Preferably, the biphasic rectangular pulse or pulse train is timed to start at the end of the T-phase of the patient's heart beat cycle.

Fig. 12A shows a spectrum of the variations of a patient's heart rate (beats per minute (bpm) typically considered over 500 R-R cycles) displayed using the display 18 of the remote controller 2. It is clearly seen from the spectrum that significant variations in the heart rate spectrum can take place even for an approximately 7 minute long measurement. The upper limit of the heart rate for the application of pulses is in this case set to 125 bpm and the lower limit is currently set to 43 bpm, these limits can naturally be varied from patient to patient using the remote controller 2.

These limits are set by the manufacturer of the stimulation unit 6. The stimulation unit 6 will not operate above the upper limit or below the lower limit. However, these limits, which are those proposed by the manufacturer, can be varied by a medical practitioner if desired. A practitioner may decide that for a particular patient it is only appropriate for that patient to receive stimulation, for example, between an upper limit of 100 bpm and a lower limit of 60 bpm.

Fig. 12B shows a spectrum of the arrhythmia associated with the heart rate spectrum of Fig. 12A. The spectrum of Fig. 12B can also be displayed on the touchscreen 18 of the remote controller 2. Arrhythmia is a medical term used for any of a large group of conditions in which there is abnormal electrical activity in the heart, i.e. when, for example, strong variations of the heart rate take place in a very limited time frame, such as is shown in Fig. 12A. The operational limits of the stimulation unit 6 in the present case are found at ∼ -25 and at ∼+ 20 in Fig. 12B, these can be varied according to the arrhythmia of the patient in question. Again, the limits are preinstalled limits.

Figs. 12A and 12B show that the remote controller 2 can be used to display parameters relative to a patient's ECG spectrum measured using the stimulation unit 6 and that the remote controller 2 is adapted to analyze the ECG spectrum in order to make a detailed analysis of the condition of the patient's heart. This analysis is typically carried out using the evaluation unit 50 of the remote controller 2.

Also shown in Figs. 12A and 12B, are three touchscreen buttons 86, 88 and 90. One of the buttons 86 is provided to start the acquisition of the next pattern, a further button 88 is provided to pause the acquisition, and the third button 90 is provided to alter the settings of the type of acquisition to take place. Moreover a fourth button 92 can be provided to display whether the requested acquisition is correct.

## Claims

1. A remote controller (2) adapted for use on a person's lap (3), the remote controller (2) having a front side (5) and a rear side (7) generally opposite to said front side (5) there being a wedge (24) at said rear side (7) generally in a central portion thereof and adapted to fit between the upper sides (9) of a person's (40) thighs when in a seated position and wherein the wedge (24) has a surface spaced from said front side (5) and inclined relative thereto such that, when the apparatus is placed on top of a horizontal surface, said front side (5) being parallel to or preferably inclined relative to the horizontal surface, wherein the remote controller (2) is a remote controller (2) for a stimulation unit (6), said stimulation unit (6) also being adapted to dock onto a docking station (4), via a docking interface (20A; 20B) to supply electrical power to a battery (60) of the stimulation unit (6) via the docking station (4), the stimulation unit (6) being adapted to communicate with the docking station (4) and/or the remote controller (2) via respective interfaces (20A, 52, 56) and the stimulation unit (6) includes at least one of a storage unit (54), an evaluation unit (56) and a microprocessor (58).

2. A remote controller (2) in accordance with claim 1, in combination with a stimulation unit (6), wherein the stimulation unit (6) is adapted to provide electrical stimulation impulses (8C, 8D, 8E, 8F) to an animal or a mammal and wherein the stimulation unit (6) is adapted to monitor (8A, 8B) a user's (40) heart rhythm or parameters related thereto such as electrical impulses originating from the mammal's heart

3. A remote controller (2) in accordance with any one of the preceding claims and having at least one interface, wherein data can be transferable via the interfaces (20A;20B;22A; 22B; 46; 52; 66;72; 74) to either the remote controller (2), the docking station (4) or the stimulation unit (6) or can be transferred to and from a remote location via the wireless interface (46; 52; 72), and/or wherein said remote controller (2) is adapted to graphically display said provided electrical stimulation impulses and/or said monitored electrical impulses.

4. A remote controller (2) in accordance with any one of claims 1 to 3, wherein the remote controller (2) includes at least one of the following, a joystick, a laptop, a keyboard, a gaming console, a push-button control and a switch control or a television remote control.

5. A remote controller (2) in accordance with any one of the preceding claims, including at least some of the following items: a battery (60), a screen, a touchscreen (18), at least one button (28, 44), at least one interface (22A, 26, 46; 20A 52, 66), at least one dial (16A, 16B, 16C, 16D), a storage unit (42; 54), an evaluation unit (50; 56), a microprocessor (48; 58), a CD-drive, a camera, a microphone, a keyboard, a DVD drive and at least one loudspeaker (42).

6. A remote controller (2) in accordance with any one of the preceding claims, wherein the electrical impulses (8C, 8D, 8E, 8F) provided by the stimulation unit (6) are biphasic generally rectangular wave impulses have a duration of approximately 1 ms to 10% of the total duration of the heart beat, have an amplitude in the range of -50 to +50 V and in particular, preferably in the range of -30 to +30V and - 45 to +45 V, and a pulse repetition frequency variable in the range from 5 Hz to 1kHz and in particular are preferably varied from 450 Hz to 1kHz.

7. A remote controller (2) in accordance with any one of the preceding claims, wherein the settings of the stimulation unit (6) can be altered by the remote controller (2), these settings being settable either by the dials (16A, 16B, 16C, 16D) provided or by data transferred uni-directionally or bidirectionally via said at least one interface (22A, 26, 46).

8. A remote controller (2) in accordance with any one of the preceding claims, wherein said settings or said data are processed by said remote controller (2) and wherein said remote controller (2) and/or said stimulation unit (6) is/are adapted to store monitored electrical impulses, and/or wherein the remote controller (2) is adapted to display parameters and/or patterns relating to the monitored electrical impulses.

9. A remote controller (2) in accordance with any one of the preceding claims further including a heart monitor, wherein if the heart monitor detects irregularities in the pattern of the heart rhythm detected, the stimulation unit (6) is adapted to transmit an emergency signal to a remote location, said signal containing information from a GPS sensor (62) and the information on the detected irregularities.

10. A remote controller (2) in accordance with any one of the preceding claims and adapted for use in combination with a patient card (26A) permitting restricted manipulation of the settings of the remote controller and for use in combination with a super user card (26A) permitting more comprehensive manipulation, said patient card (26A) and said super user card (26A) being insertable into a card interface (26, 76).

11. A remote controller (2) in accordance with any one of the preceding claims, having at least one of the following elements, a credit meter (47; 63; 84), a tablet dispenser, a diary, a routine monitor.
